# EUROPEAN PATENT APPLICATION

(11) **EP 0 789 072 A2**
(43) Date of publication of application: **13.08.1997**
(21) Application number: 96111432.9
(22) Date of filing: 16.07.1996
(51) Int. Cl.: C12M 1/24, C12M 3/04

(54) **Growth environment assembly and method of use therof**

(30) Priority: 15.08.1995 US 515310
(71) Applicant: Becton Dickinson and Company, Franklin Lakes, New Jersey 07417-1880 (US)
(72) Inventor: O'Leary, Robert K., Morris Plains, N.J. 07950 (US); LaRocca, Paul J., Pepperell, Massachusetts 001463 (US); Stevens, Timothy A., Warwick, New York 10990 (US)
(74) Representative: Selting, Günther, Dipl.-Ing.

(57) **Abstract**

A method and assembly for treating living and non-living biological environments with varying doses of fluids, solutes, drug molecules or diffusible agents *in vitro*. The assembly comprises a vessel, a closure, and a sustained release matrix material located on the bottom inside the vessel. The assembly provides means for carrying out pharmacokinetic and toxicokinetic studies.

## Description

### BACKGROUND OF THE INVENTION

### 1. Field of the Invention

This invention relates to a method and assembly for treating living and non-living biological environments with varying doses of fluids, solutes, drug molecules or diffusible agents *in vitro,* and more particularly to a vessel and closure assembly having means for releasing fluids, solutes, drug molecules or diffusible agents in environments that may include cell lines and tissue cultures.

### 2. Description of Related Art

The use of *in vitro* cell lines and tissue cultures in carrying out pharmacokinetic and toxicokinetic studies related to safety evaluations of new drugs is a growing area of scientific development. By using cell lines and tissue cultures, the need for large animal studies is reduced.

Pharmacokinetic and toxicokinetic studies depend upon the control rate of drug delivery and dose of drug to a cell line for multiple drugs simultaneously. Repeated drug administration can influence the toxicity of the drug by changing its metabolism and stimulating the cell's synthesis of certain proteins that can effect the drug's activity on the cell line.

Current technologies for studying *in vitro* pharmacokinetics and toxicokinetics use either a closed monolayer culture system (MCS) or a dynamic perfused cell system (DPCS). The MCS employs an immediate total dose application of the test drug. Its limitation is that it operates in essentially a single dose range which can be toxic due to over dose.

On the other hand, the DPCS acts as an *in vivo* circulating model. The in *vitro* route of bolus administration does not mimic the *in vivo* method of drug administration since their are no tissue masses to act as the sustain release matrix.

A need exists for a tissue culture drug/biological delivery and/or releasing system in order to carry out *in vitro* pharmacokinetic metabolic pathway studies of drugs, proteins, growth factors and other such biologicals or growth factors. The need to deliver and/or release a perspective drug to cell lines requires a controlled and continuous rate of delivery to the biological environment. The need arises because often it is experimentally undesirable to deliver fluids, such as drugs to cells and/or tissue cultures in bolus concentrations. Bolus concentrations cause the pharmacological dose to quickly and uncontrollably extend into the toxicological range. Bolus delivery by comparison to a linear sustain release delivery closely mimics the ultimate clinical experience in the practice of medicine.

Therefore, a special need exists for a drug delivery and/or releasing system which permits the control of drug delivery and/or release and provides an assessment of drug concentration on cell lines and tissue culture environments so as to establish a correlation of drug action with adverse reactions.

Such a need for a new type of drug delivery and/or releasing system for treating biological environments has not been suggested or taught in the literature.

### SUMMARY OF THE INVENTION

The present invention is a method and assembly for delivery and/or releasing fluids, solutes, drug molecules or diffusible agents in environments, such as living and non-living biological environments that may include such as cell lines and tissue cultures.

Most preferably, the present invention is an assembly comprising a vessel and closure. Preferably, the vessel comprises means for releasing fluids, such as drugs, proteins, growth factors and other such biologicals into the vessel. Most preferably, the means for releasing fluids into the vessel is a sustained release matrix material removably attached inside the vessel.

The material is removably attached inside the vessel in such a way to allow the releasing of at least one drug to cells in the vessel. The sustained release matrix material is activated by a concentration gradient according to Fick's First Law.

Preferably, the vessel is a flask, roller bottle, tube, spinner flask, stirred bioreactor or any vessel that will facilitate cell culture viability. Most preferably, the vessel is a flask or roller bottle. Vessels that are applicable to this invention include those described in U.S. Patent Nos. 5,272,084; 4,770,854; 5,139,952; 4,334,028; 4,289,248; 4,387,822; and 5,047,347, which are incorporated by reference.

Desirably, the closure is a cap, push cap, threaded cap, screw cap or stopper.

Most notably, the assembly of the present invention provides a continuous controlled release of fluids or solute drug molecules which thereafter diffuse from the sustain release matrix material and to the cells. The present invention substantially eliminates the delivery of bolus concentrations of fluids which causes the pharmacological dose to quickly and uncontrollably extend into the toxicological range.

In a preferred alternate embodiment, the present invention further comprises means for delivering additional fluids or molecules such as drugs, proteins, growth factors and other such biologicals into the vessel. Most preferably, the means for delivery additional fluids into the vessel is a tubular membrane extending from the closure and suspended into the vessel.

The tubular membrane is preferably attached to the closure in such a way to allow the membrane to be easily rotated inside the vessel. The tubular membrane is rotated as fluid is being delivered so as to eliminate the media protein boundary layer resistance at the membrane surface. The ability to rotate the tubular membrane also facilities stirring of cell media in the vessel, therefore, permitting linear steady state kinetics of drug diffusion to proceed.

In addition, the top of the closure may preferably comprise an entry port from which the tubular membrane extends into the vessel. The tubular membrane comprises an open end and a closed end, wherein the open end is connected to the entry port of the closure and the closed end extends into the vessel. The tubular membrane comprises a hollow cavity containing the means to distribute substances.

Most preferably the tubular membrane is tangentially attached to the closure and its mobility inside the vessel is brought about by rotating of the closure. This rotating motion of the tubular membrane provides bi-directional rotation at variable speed so as to produce less media protein boundary layer resistance at the outer surface of the tubular membrane. Protein boundary layers are complex and are heterogenous protein films which are readily deposited on membranes. The proteins are substantially released by turbulence at the surface of the membrane created by the rotating motion of the membrane and the closure.

The tubular membrane is an imperfect barrier separating two fluids and hinders free diffusion of a substance in an isotropic medium. Diffusion is the tendency of molecules to migrate from a region of high concentration (potential and/or activity) to a region of lower concentration (potential and/or activity).

The tubular membrane has the capability of delivering microspikes of drug when the membrane is rapidly rotated because the static diffusivity of the drug in the membrane is increased to a dynamic diffusivity because the exiting drug is carried away from the surface of the membrane thus eliminating back diffusion or stagnation at the outer surface of the membrane. Thus a new concentration gradient is established at the outer surface of the membrane. Sudden changes in the slope of the drug release curve such that the slope of release increases m a positive direction, and is quickly changed in a negative direction over a short time interval typically not exceeding a one to three minute total interval. A microspike delivers less than one percent of the total potential diffusate over the full course of diffusion.

An alternate embodiment of the assembly of present invention may comprise multiple tubular membranes attached to the closure and extending into the vessel so that more than one drug solute may be injected into the tubular membrane at one time or at varying time intervals.

The use of die assembly of the present invention permits the control of fluid, solute or diffusible agents delivery and concentration to cell lines and tissue cultures and permits the development of steady state drug diffusion for studying possible mechanisms for the passage of nutrients and drugs and for studying *in vitro* pharmacokinetic studies on living cells.

The present invention employs a sustained release surface matrix and/or a hollow fiber system for sustained drug release and/or delivery and as such is a good model for studying drug dose response curves on tissue slices and/or cell populations and organ cultures, bacterial fungi, microorganisms or viral cultures.

The present invention provides important advances over methods used to perform pharmacokinetic and toxicokinetic studies. The assembly of the present invention provides the ability to maintain an *in vitro* culture system wherein the cells are subjected to an environment which permits them to function in a manner closely simulating that encountered *in vivo.* The invention also permits the efficient removal of desired metabolic products from the cells being cultured and provides important advantages in the efficiency, economy and flexibility of operation.

The present invention provides a leak-proof, self-contained environment for the use and safe disposal of infected cultures and dangerous toxicants.

The present invention can also be used for fluorescent tracing of drugs, radiolabeling and radiographic imaging of drugs and for supplementing basal media requirements with, for example, growth factors and hormones.

A further advantage of the present invention include its use in drug therapy by providing quantitative evaluations of cell populations responding to drug therapy. Whereby, kinetic rate constants, activation energies, entropies and other basic pharmacological parameters can be evaluated. Another feature of the present invention is that metabolic substances such as uremic poisons can be delivered to cells for the purpose of investigating the effects of metabolites on *in vitro* cultured cells.

### DESCRIPTION OF THE DRAWINGS

FIG. 1 is a perspective view of the assembly of the present invention.

FIG. 2 is a perspective view of the cap of FIG. 1.

FIG. 3 is a top view of the cap of FIG. 2.

FIG. 4 is a perspective view of an alternate cap embodiment of the present invention.

FIG. 5 is a perspective view of an alternate cap embodiment of the present invention.

FIG. 6 illustrates the kinetics of a typical drug release from a sustained matrix material into a tissue culture flask.

### DETAILED DESCRIPTION

While this invention is satisfied by embodiments in many different forms, there is shown in the drawings and will herein be described in detail, the preferred embodiments of the invention, with the understanding that the present disclosure is to be considered as exemplary of the principles of the invention and is not intended to limit the invention to the embodiments illustrated. Various other modifications will be apparent to and readily made by those skilled in the art without departing from the scope and spirit of the invention. The scope of the invention will be measured by the appended claims and their equivalents.

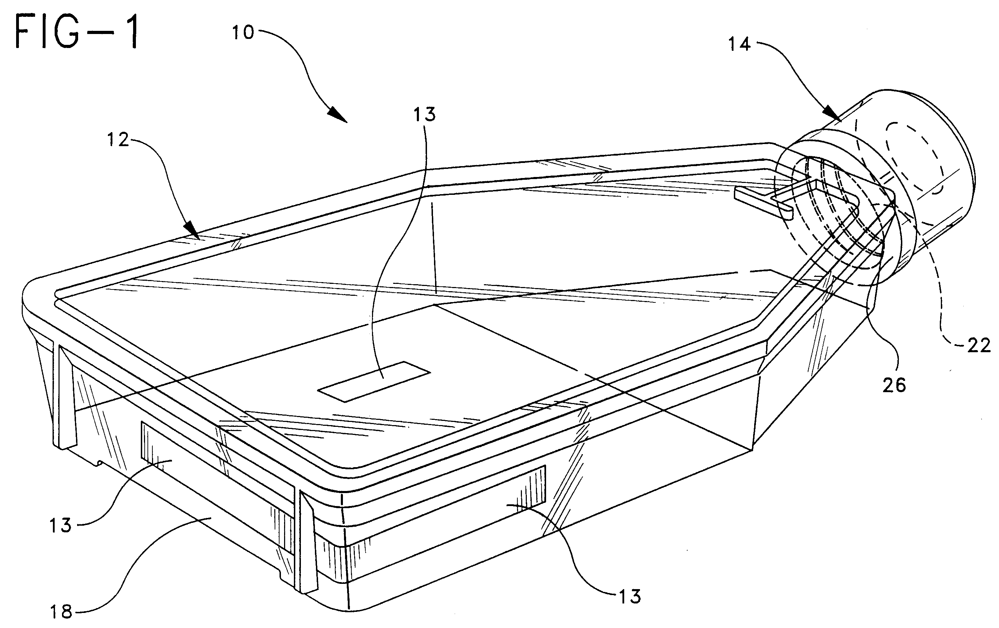

FIG. 1 illustrates an assembly **10** of the present invention, comprising a vessel **12**, a closure **14** and at least one sustained release matrix material **13**. The vessel is preferably made from impact resistant plastic or glass which is gas impermeable, optically clear, non-toxic and inert with respect to the cells to be cultured.

Sustained release matrix material **13** may be made from polymers including both synthetic and natural matrices having permeability coefficients for drugs that lead to diffusion of bioactive concentrations. Preferably, the sustained released matrix material is made from low molecular weight polyorthoesters, collagen, human bone, fried egg or the like.

The sustained release matrix material may be any shape or size and be situated inside the vessel at any desirable location.

Vessel **12** has a body **18** in which material is adapted to be held until such time as the same is withdrawn or dispensed. It is unimportant whether body **18** is made of a collapsible or non-collapsible material, such as metals, plastics or glass.

As shown in FIG. 1, vessel **12** includes a neck **22** to receive closure **14**. Neck **22** is integral with the vessel and defines a cylindrical conduit having one end integral with the vessel and the other end defining an opening through which the cells and culture fluids may be introduced into the body of the vessel. Neck **22** and closure **12** constitute one of a number of well known means for introducing materials such as mammalian cells and culture fluids into body **18**. As is conventionally know, closure **14** is removed from neck **22** to provide an opening through which cells and culturing fluids can be introduced into the vessel. The closure is subsequently remounted onto the neck to re-seal the vessel.

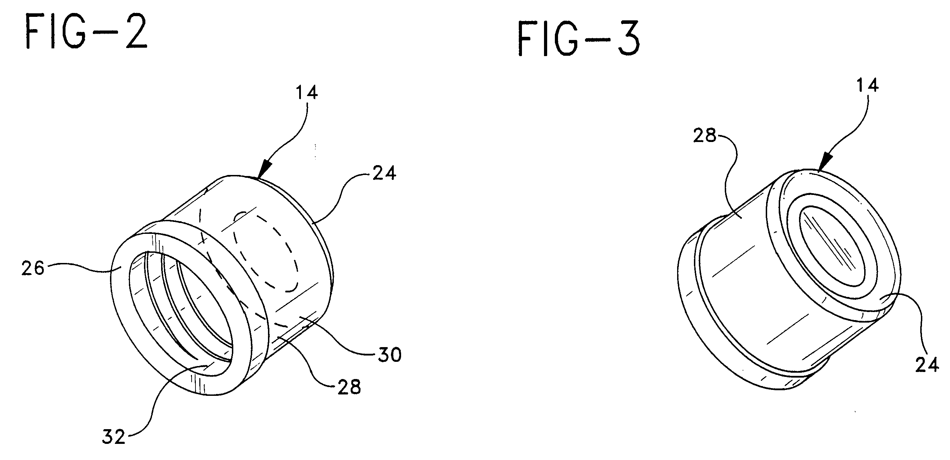

As shown in FIGS. 2 and 3, closure **14** has a top surface **24**, a bottom stop ledge **26** and an annular outer skirt **28** extending from the top surface to the bottom stop ledge. The annular outer skirt has an outer wall surface **30** and an inner wall surface **32**.

Alternate embodiments of the closure of the present invention are shown in FIGS. 4-5 and include many components which are substantially identical to the components of FIGS. 2-3. Accordingly, similar components performing similar functions will be numbered identically to those components of FIGS. 2-3, except that a suffix "a" will be used to identify those similar components in FIGS. 4-5.

Closure **14a** as shown in FIG. 4 is an alternate embodiment of the closure that can be used with vessel **12**. The top surface of the closure further comprises entry port **34** and sampling port **36**. A tubular membrane **16** extends from entry port **34** of the closure and into the vessel. As shown in FIG. 4, tubular membrane **16** comprises an open end **38**, a closed end **40** and a hollow cavity **42** containing the means to distribute fluid substances. Tubular membrane **16** comprises a sidewall **44** that extends from open end **38** to closed end **40** and comprises an inner surface **46** and outer surface **48**.

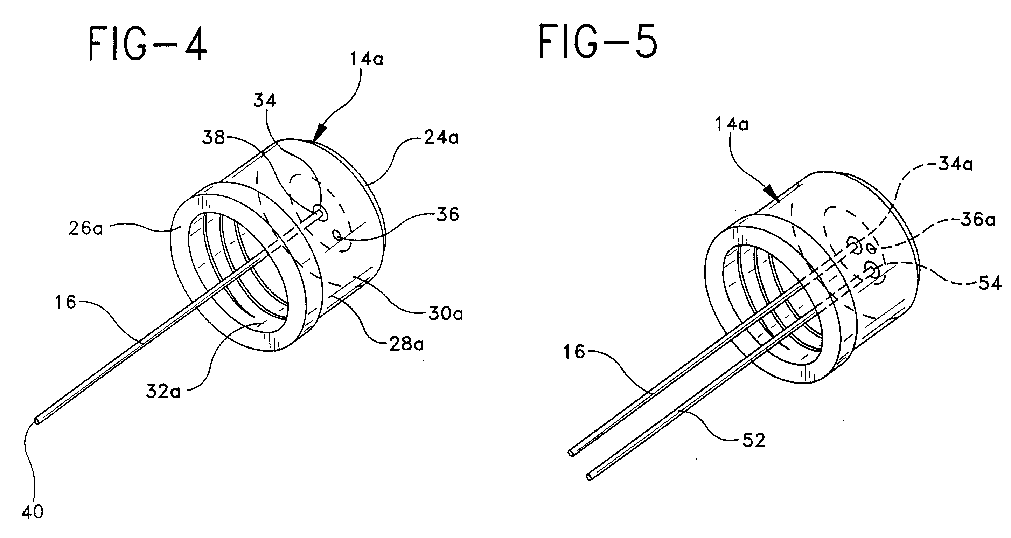

Another alternate embodiment of the present invention is shown in FIG. 5. The alternate embodiment is a second tubular membrane **52** attached to the top of the closure at a second entry port **54** and extending into the vessel. Although FIG. 5 only shows two tubular membranes, it is well within the purview of the invention to have more than two tubular membranes extending into the vessel and/or attached to the top of the closure.

Most preferably, the top surface of the closure is a pierceable self sealing material whereby entry can be made into the tubular membrane through entry port **34** or into the vessel by sampling port **36** by means for example by a hypodermic needle so that fluids can be injected into the tubular membrane and/or added or removed from the vessel without breaking the environment of the vessel. After the needle is removed, the self sealing material immediately reseals. The material is most preferably an elastomeric material.

Tubular membrane **16** may be made from any suitable gas permeable material so long as it provides for the passage of fluids or molecules such as drugs and biological fluids into vessel **12**. Preferably, the tubular membrane is made from a natural or synthetic polymer or a dialysis material.

Diffusion is characterized by the tendency of molecules to migrate from a region of high concentration to a region of lower concentration, but is more rigorously stated with respect to chemical potentials or activities rather than concentrations. The membrane is an imperfect barrier separating two fluids and hinders free diffusion of a substance in an isotropic medium.

Dialysis is due to sieve action and is largely dependent on the molecular weight of the diffusate and the viscosity of the solvent in accordance with the Sutherland and Einstein equation. Dialysis membranes may be considered as heterogeneous barriers in the sense that they possess pores. The ease of transport is generally a measure of the probability of a solvated molecule entering and diffusing through these pores. There is little selectivity in the separation of two closely related molecules except when their size is approximately that of the size of the pore. In general, the solvent, as well as the solute is transported; membranes which allow salt transport are permeable to water.

The diameter of the tubular membrane is about 150 micron to about 300 micron and most preferably about 150 microns um. The length of the tubular membrane is dependent on the size of the vessel being used.

Priming volumes of about 0.25 ml to about 2.5 ml with macro fibers of about 2.5 ml to about 5.0 ml

The solute permeability of the tubular membrane may be small, medium or large. An example of the solute permeability are as follows: (i) a small solute permeability is about 50 to about 5000 molecular weight (i.e., urea, or insulin); (ii) a medium size solute permeability is about 6000 to about 37,000 molecular weight (i.e., insulin, or heparin); and (iii) a large molecule permeability is about 150,000 antibodies.

Multiple drugs can be delivered simultaneously or sequenced for agonist or antagonistic effects. Typical uses could be changes in parameters such as temperature, different cells organisms, different gaseous environments, different media and additives.

The injectable feature of the tubular membrane permits the investigator to alter the kinetics of the tubular membrane diffusivity by pre-injecting the tubular membrane with surfactants or other solvents such as dimethyl sulfoxide to accelerate or control the drug diffusion process.

The tubular membrane may be used to distribute substances such as drugs, hormones, insecticides, phomones and repellents into the vessel. The tubular membrane provides a means for the controlled release of the active substances wherein the substrate consists of an asymmetric wall formed from synthetic or natural polymers.

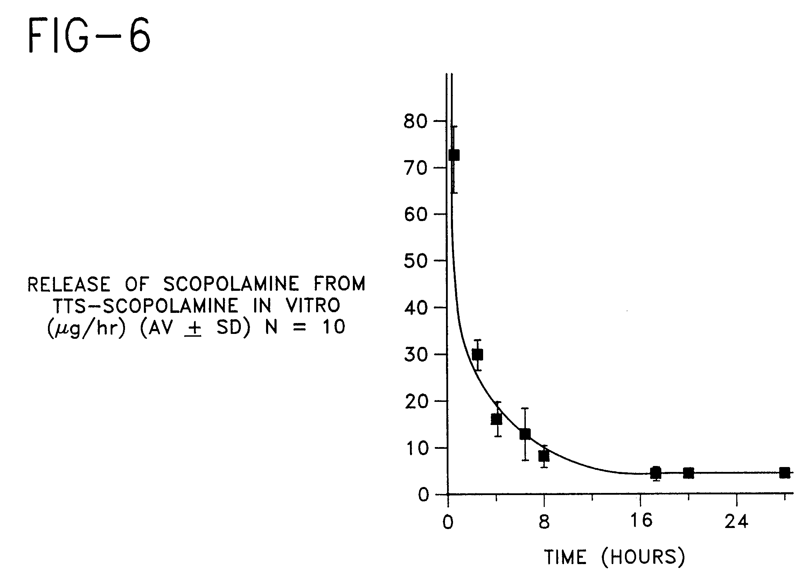

Figure 6 illustrates the kinetics of a controlled drug administration model suitable for use in tissue culture and in particular with the present invention. This model depicts the release of a typical drug from a matrix over time. The initial drug concentration is high prior to its release into the surrounding environment. The actual drug release follows a linear log-Fickian diffusion process at constant temperature. The diffusion rate becomes linear followed by a tapered decrease in the later phase of drug release.

## Claims

1. An assembly for treating living and non-living biological environments comprising:
a vessel comprising a chamber and a neck connected to said chamber having an opening for introducing cells and culture fluids into said chamber;
a closure for covering said opening in said neck comprising means for removably mounting said closure to said neck; and
means for releasing fluids in said vessel.

2. The assembly of Claim 1 wherein said means for releasing fluids in said vessel is a sustained release matrix material located in said vessel.

3. The assembly of Claim 2 wherein said closure comprises a top portion, a bottom portion, an annular skirt extending from said top portion to said bottom portion and having an inner surface and outer surface.

4. The assembly of Claim 3 further comprising an entry port and at least one tubular membrane connected to said entry port for delivering fluids into said vessel.

5. The assembly of Claim 4 wherein said tubular membrane comprises an open end, a closed end and a hollow cavity.

6. The assembly of Claim 1 wherein said vessel is a flask or a roller bottle.

7. The assembly of Claim 3 wherein said closure and said tubular membrane are rotated to reduce boundary layer resistance to fluid diffusion and to facilitate stirring of fluids in said vessel.

8. The assembly of Claim 3 wherein said top portion of said closure is a pierceable self sealing material.

9. The assembly of Claim 3 wherein said top portion of said closure further comprises a sampling port.

10. The assembly of Claim 1 wherein said means for delivering fluids into said vessel is at least two tubular membranes.
